# EUROPEAN PATENT APPLICATION

(11) **EP 4 775 121 A1**
(43) Date of publication of application: **15.07.2026**
(21) Application number: 25151105.1
(22) Date of filing: 10.01.2025
(51) Int. Cl.: A61B 1/00, A61B 3/00, A61B 3/10, A61B 3/12, A61B 3/14, G06T 7/00

(54) **PATIENT ALIGNMENT SYSTEM FOR AN OPHTHALMIC IMAGING INSTRUMENT**

(71) Applicant: Optos plc, Dunfermline, Scotland KY11 8GR (GB)
(72) Inventor: EASTWOOD, Richard, Dunfermline, Scotland, KY11 8GR (GB)
(74) Representative: Hoffmann Eitle

(57) **Abstract**

An ophthalmic imaging instrument which acquires an image of a fundus of an eye, comprising: an ellipsoidal mirror having a first focal point and a second focal point, wherein a pupil of the eye is at the second focal point when the instrument images the fundus via the ellipsoidal mirror; a stereo imaging apparatus which acquires stereo images of the pupil by detecting light reflected by the ellipsoidal mirror via the first focal point; and a processor which generates, based on the stereo images, a signal for bringing the pupil within a target range for acquiring the image by: generating dewarped stereo images of the pupil by compensating for a warping in the stereo images caused by the ellipsoidal mirror; and processing the dewarped stereo images to generate the signal.

## Description

### [Field]

Example aspects herein generally relate to the field of ophthalmic imaging instruments and, more particularly, to systems for bringing an eye of a subject into a position for acquiring an image of a fundus of the eye by an ophthalmic imaging instrument.

### [Background]

Ophthalmic imaging instruments employ various techniques to image different parts of an eye of a subject, and are used by clinicians to diagnose and manage a variety of eye conditions. For example, ophthalmic imaging scanners acquire images of the fundus or other part of the eye by scanning a beam of light across the eye and detecting return light from the eye. To acquire high quality images of the fundus of the eye, ophthalmic imaging scanners, such as scanning laser ophthalmoscopes (SLOs) and optical coherence tomography (OCT) scanners, for example, often require the position of an exit pupil of the ophthalmic imaging scanner to fall within a predetermined range of distances from the pupil of the eye that are suitable for acquiring such images. Pupil alignment can be particularly important for widefield (WF) and ultra-widefield (UWF) ophthalmic imaging instruments, where the acquisition of a WF or UWF fundus image relies on light propagating to and from the fundus, through the pupil, at widely varying angles of incidence on the pupillary plane, which is prone to being clipped by the pupil unless the pupil alignment is satisfactory.

Stereoscopic ranging techniques employing stereo cameras are often used to measure distance to the eye. For example, some ophthalmic imaging instruments include a so-called pupil alignment module (PAM), which comprises stereo cameras to acquire stereo images of the eye, and is arranged to locate respective pupil centres in the stereo images, and determine the distance between the PAM and the pupil based on a separation between the located pupil centres in the stereo images. Visual feedback based on the determined distance may be provided to guide the subject in bringing their eye into a position suitable for imaging the eye.

The stereo cameras of a PAM are typically mounted on the ophthalmic imaging instrument to have a direct visual pathway to the subject's eye (i.e. with there being no intervening optical element). However, in mirror-based ophthalmic imaging scanner, particularly a WF or UWF ophthalmic imaging scanner, the mirror-based optics which typically employ an ellipsoidal mirror to transfer the scan to/from the eye, can make the provision of such a direct visual pathway difficult or impossible. The alternative of guiding light from the eye to the stereo cameras using additional optical elements within the mirror-based optics can increase the complexity of the system and present significant engineering challenges, for example in dealing with spurious reflections, which may be caused by the additional optical elements, and which may manifest as artifacts in the acquired images.

### [Summary]

There is provided, in accordance with a first example aspect herein, an ophthalmic imaging instrument arranged to acquire an image of a fundus of an eye, comprising: an ellipsoidal mirror, via which the ophthalmic imaging instrument is arranged to acquire the image of the fundus, the ellipsoidal mirror having a first focal point and a second focal point, wherein a pupil of the eye is positioned at the second focal point during use of the ophthalmic imaging instrument; a stereo imaging apparatus arranged to acquire stereo images of the pupil by detecting light from the eye which has been reflected by the ellipsoidal mirror via the first focal point of the ellipsoidal mirror; and a processor. The processor is arranged to generate, based on the stereo images of the pupil, a signal for bringing the pupil of the eye within a target range for acquiring an image of the fundus by: generating dewarped stereo images of the pupil by processing the stereo images to compensate for a warping in the stereo images caused by a curvature of the ellipsoidal mirror; and processing the dewarped stereo images to generate the signal for bringing the pupil of the eye within the target range.

In some example embodiments, the processor is arranged to process the dewarped stereo images to generate the signal by: processing each image of the dewarped stereo images to locate a respective pupil centre of the pupil in the image; determining a separation between the pupil centres in a common image frame; using the separation to determine an indication of a distance between the pupil of the eye and a reference position of the ophthalmic imaging instrument at which the pupil is to be placed for acquiring the image of the fundus; comparing the indication of the distance with at least one pre-determined threshold; and generating the signal using a result of the comparing. The processor may be arranged to process each image of the dewarped stereo images to locate the respective pupil centre of the pupil in the image by using edge detection to determine an outline of the pupil in the image, and fitting a circle to the outline to determine, as the pupil centre of the pupil in the image, a centre of the circle.

In the ophthalmic imaging instrument of the first example aspect or the example embodiments set out above, the processor may, in accordance with an example embodiment, be arranged to generate the dewarped stereo images of the pupil by using a mapping, which is based on an optical model of the ellipsoidal mirror, to transform the stereo images to compensate for the warping in the stereo images caused by the curvature of the ellipsoidal mirror.

In the ophthalmic imaging instrument of the first example aspect or any of the example embodiments set out above, the ophthalmic imaging instrument may, in accordance with some example embodiments, be arranged to acquire the image of the fundus by scanning a beam of light across the fundus, and further comprise: a light source arranged to emit the beam of light; and a rotatable mirror arranged to scan the beam of light across the fundus via the first focal point of the ellipsoidal mirror and via the ellipsoidal mirror. Furthermore, the stereo imaging apparatus may be arranged to acquire the stereo images of the pupil via the rotatable mirror and the ellipsoidal mirror, and the processor may be arranged to operate in a patient alignment mode to generate the signal, and to subsequently operate in a fundus imaging mode to control the ophthalmic imaging instrument to acquire the image of the fundus. In the patient alignment mode, the processor may be arranged to: control the rotatable mirror to be stationary in a predetermined orientation; and control the stereo imaging apparatus to acquire the stereo images of the pupil while the rotatable mirror is stationary in the predetermined orientation. In the fundus imaging mode, the processor may be arranged to: control the light source to emit the beam of light; and control the ophthalmic imaging instrument to acquire the image of the fundus by controlling the rotatable mirror to scan the beam of light across the fundus via the ellipsoidal mirror.

In any of the example embodiments set out above, where the ophthalmic imaging instrument is arranged to acquire the image of the fundus by scanning a beam of light across the fundus and further comprises the light source and the rotatable mirror, the stereo imaging apparatus may be located within a region between the rotatable mirror and the ellipsoidal mirror not traversed by the beam of light during acquisition of the image of the fundus. The predetermined orientation of the rotatable mirror may be such that the rotatable mirror starts rotating from the predetermined orientation when the ophthalmic imaging instrument starts acquiring the image of the fundus. Additionally or alternatively, the stereo imaging apparatus may be located between the rotatable mirror and the ellipsoidal mirror at a location which minimises a deviation from circularity of respective representations of the pupil in the stereo images of the pupil acquired by the stereo imaging apparatus when the rotatable mirror is in the predetermined orientation. In some example embodiments, the ellipsoidal mirror may have a plane of symmetry comprising the first focal point and the second focal point, a normal direction of the rotatable mirror at the first focal point may subtend a predetermined angle relative to the plane of symmetry when the rotatable mirror is in the predetermined orientation, and the stereo imaging apparatus may comprise a first camera and a second camera, wherein the first camera and the second camera are arranged equidistantly from and on opposite sides of a second plane comprising the first focal point and the second focal point, and wherein the normal direction of the rotatable mirror and the second plane subtend an angle at the first focal point which is less than two times as large as the predetermined angle. In these example embodiments, the first camera may have a first optical axis, and the second camera may have a second optical axis which is parallel to the first optical axis, and the stereo imaging apparatus may further comprise a Fresnel lens disposed between the rotatable mirror and both the first camera and the second camera, the Fresnel lens being arranged to refract light from the eye, which has been reflected by the rotatable mirror (e.g. at the first focal point of the ellipsoidal mirror), to propagate along the first optical axis and the second optical axis. In some other example embodiments, the stereo imaging apparatus may comprise a first camera having a first optical axis, a second camera having a second optical axis which is parallel to the first optical axis, and a Fresnel lens disposed between the rotatable mirror and both the first camera and the second camera, the Fresnel lens being arranged to refract light from the eye, which has been reflected by the rotatable mirror (e.g. at the first focal point of the ellipsoidal mirror, in case the rotatable mirror is arranged to reflect the beam of light at the first focal point), to propagate along the first optical axis and the second optical axis.

In any of example embodiments set out above, where the ophthalmic imaging instrument is arranged to acquire the image of the fundus by scanning a beam of light across the fundus and further comprises the light source and the rotatable mirror, the stereo imaging apparatus may comprise: a first camera having a first optical axis, and a second camera having a second optical axis which is parallel to the first optical axis; and a Fresnel lens disposed between the rotatable mirror and both the first camera and the second camera, the Fresnel lens being arranged to refract light from the eye, which has been reflected by the rotatable mirror, to propagate along the first optical axis and the second optical axis.

In any of example embodiments set out above, where the ophthalmic imaging instrument is arranged to acquire the image of the fundus by scanning a beam of light across the fundus and further comprises the light source and the rotatable mirror, the ophthalmic imaging instrument may further comprise a fixation target light source arranged to project fixation light onto the fundus via the rotatable mirror and the ellipsoidal mirror, wherein, in the patient alignment mode, the processor is arranged to control the fixation target light source to project the fixation light onto the rotatable mirror in the predetermined orientation, the fixation target light source being disposed in relation to the rotatable mirror such that the fixation light is projected onto the fundus via the ellipsoidal mirror to fix a gaze direction of the eye. The fixation target light source may be located within the region mentioned above, which is between the rotatable mirror and the ellipsoidal mirror not traversed by the beam of light during acquisition of the image of the fundus. The predetermined orientation may be such that the rotatable mirror starts rotating from the predetermined orientation when the ophthalmic imaging instrument starts acquiring the image of the fundus. For example, the fixation target light source may be arranged to project the fixation light onto the rotatable mirror from a location such that, when the rotatable mirror is in the predetermined orientation, the fixation light is incident on the eye in a direction for central gaze fixation of the eye.

In the ophthalmic imaging instrument of the first example aspect or any of its example embodiments set out above, the signal for bringing the pupil of the eye within the target range for acquiring the image of the fundus may comprise: a projection of light of a first colour (e.g. blue) onto the eye in case the subject is to move the eye towards the ophthalmic imaging instrument to bring the pupil towards the target range; a projection of light of a second colour (e.g. red) onto the eye in case the subject is to move the eye away from the ophthalmic imaging instrument to bring the pupil towards the target range; and a projection of light of a third colour (e.g. green) onto the eye in case the pupil is within the target range suitable for acquiring the image, wherein the first colour, the second colour and the third colour are different from one another. Additionally or alternatively, the signal for bringing the pupil of the eye within the target range for acquiring the image of the fundus may comprise: a first sound (e.g. tone or spoken word(s)) in case the subject is to move the eye towards the ophthalmic imaging instrument to bring the pupil towards the target range; a second sound (e.g. tone or spoken word(s)) in case the subject is to move the eye away from the ophthalmic imaging instrument to bring the pupil towards the target range; and a third sound (e.g. tone or spoken word(s)) in case the pupil is within the target range suitable for acquiring the image, wherein the first sound, the second sound and the third sound are different from one another.

The ophthalmic imaging instrument of the first example aspect or any of its example embodiments set out above may comprise an illumination source arranged to illuminate the eye via the rotatable mirror and the ellipsoidal mirror with light for acquiring the stereo images of the pupil by the stereo imaging apparatus.

The ophthalmic imaging instrument of the first example aspect or any of its example embodiments set out above may be an UWF ophthalmic imaging instrument arranged to acquire an UWF image of the fundus, or a WF ophthalmic imaging instrument arranged to acquire a WF Image of the fundus, for example. The ophthalmic imaging instrument may comprise at least one of an UWF SLO or an UWF OCT imaging instrument in some example embodiments.

There is provided, in accordance with a second example aspect herein, a method of generating a signal for bringing a pupil of an eye within a target range for acquiring an image of a fundus of the eye by an ophthalmic imaging instrument, the ophthalmic imaging instrument comprising: an ellipsoidal mirror, via which the ophthalmic imaging instrument is arranged to acquire the image of the fundus, the ellipsoidal mirror having a first focal point and a conjugate second focal point, wherein the pupil of the eye is positioned at the second focal point during use of the ophthalmic imaging instrument; and a stereo imaging apparatus arranged to acquire stereo images of the pupil by detecting light from the eye which has been reflected by the ellipsoidal mirror via the first focal point of the ellipsoidal mirror. The method comprises: controlling the stereo imaging apparatus to acquire the stereo images of the pupil via the first focal point of the ellipsoidal mirror; generating dewarped stereo images of the pupil by processing the stereo images to compensate for a warping in the stereo images caused by a curvature of the ellipsoidal mirror; and processing the dewarped stereo images to generate the signal for bringing the pupil of the eye within the target range.

In some example embodiments, the dewarped stereo images are processed to generate the signal by: processing each image of the dewarped stereo images to locate a respective pupil centre of the pupil in the image; determining a separation between the pupil centres in a common image frame; using the separation to determine an indication of a distance between the pupil of the eye and a reference position of the ophthalmic imaging instrument at which the pupil is to be placed for acquiring the image of the fundus; comparing the indication of the distance with at least one pre-determined threshold; and generating the signal using a result of the comparing. In these example embodiments, each image of the dewarped stereo images may be processed to locate the respective pupil centre of the pupil in the image by using edge detection to determine an outline of the pupil in the image, and fitting a circle to the outline to determine, as the pupil centre in the image, a centre of the circle.

In the method of the second example aspect or any of the embodiments set our above, the dewarped stereo images of the pupil may be generated by using a mapping, which is based on an optical model of the ellipsoidal mirror, to transform the stereo images to compensate for the warping in the stereo images caused by the curvature of the ellipsoidal mirror.

In the method of the second example aspect or any of its example embodiments set out above, the ophthalmic imaging instrument may be an UWF ophthalmic imaging instrument arranged to acquire an UWF image of the fundus, or a WF ophthalmic imaging instrument arranged to acquire a WF Image of the fundus, for example. The ophthalmic imaging instrument may comprise at least one of an UWF SLO or an UWF OCT imaging instrument in some example embodiments.

There is also provided, in accordance with a third example aspect herein, a computer program comprising computer-readable instructions that, when executed by the processor of the ophthalmic imaging instrument of the first example aspect or any of its example embodiments set out above, cause the processor to perform the method of the second example aspect or any of its embodiments set out above. The computer program may be stored on a non-transitory computer-readable storage medium (such as a computer hard disk or a CD, for example) or carried by a computer-readable signal.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ultra-widefield ophthalmic imaging instrument according to an example embodiment herein.
Figure 2 is a schematic illustration of a programmable signal processing hardware, which may be configured to perform the functions of the processor 50 described herein.
Figure 3A is a schematic illustration of a warped image of the imaging target shown in Figure 3B.
Figure 3B is a schematic illustration of an example imaging target which may be used to determine a mapping for de-warping images acquired by the ultra-widefield ophthalmic imaging instrument according of the example embodiment.
Figure 4 is schematic showing a cross-section of the ellipsoidal mirror 32 of the example embodiment in a plane which is perpendicular to the axis of symmetry of the ellipsoidal mirror, as well as top view of the stereo imaging apparatus and fixation target light source of the example embodiment, along the axis of symmetry.
Figure 5 is a schematic illustration of an ultra-widefield ophthalmic imaging instrument according to a variant of the example embodiment herein, which comprises two ellipsoidal mirrors having a shared focal point, wherein the rotating mirror is provided at the remaining focal point of one of the ellipsoidal mirrors to scan line-field illumination across the fundus via the pupil provided at the remaining focal point of the other ellipsoidal mirror.
Figure 6 is a flow diagram illustrating a method of generating a signal for bringing a pupil of an eye within a target range for acquiring an ultra-widefield image of a fundus of the eye by the ultra-widefield ophthalmic imaging instrument of the example embodiment.
Figure 7 is a flow diagram illustrating an example implementation of S30 in Figure 6.

### [Detailed Description of Example Embodiments]

There are described herein arrangements of a stereo imaging apparatus for acquiring stereo imaging of the pupil within a mirror-based WF or UWF ophthalmic imaging instrument that at least partially address one or more of the problems set out above. In these arrangements, the imaging instrument is arranged to acquire the WF or UWF images of a fundus of an eye via an ellipsoidal mirror, and a stereo imaging apparatus is provided to acquire stereo images of a pupil of the eye also via the ellipsoidal mirror, which are used to generate a signal for bringing the pupil within a target range for acquiring the WF or UWF image of the fundus. The inventors have found that a warping in the stereo images caused by a curvature of the ellipsoidal mirror, although usually small and typically ignored, can have a surprisingly large effect on the accuracy with which pupil centres can be determined in the stereo images, and therefore the accuracy with which the pupil may be aligned for acquisition of the image of the ocular fundus using stereoscopic ranging techniques. The inventors have found that significantly improved pupil alignment, which is particularly important for WF and UWF ophthalmic imaging instruments, as discussed below, may be achieved by de-warping the stereo images before using them to generate the signal for bringing the pupil within the target range.

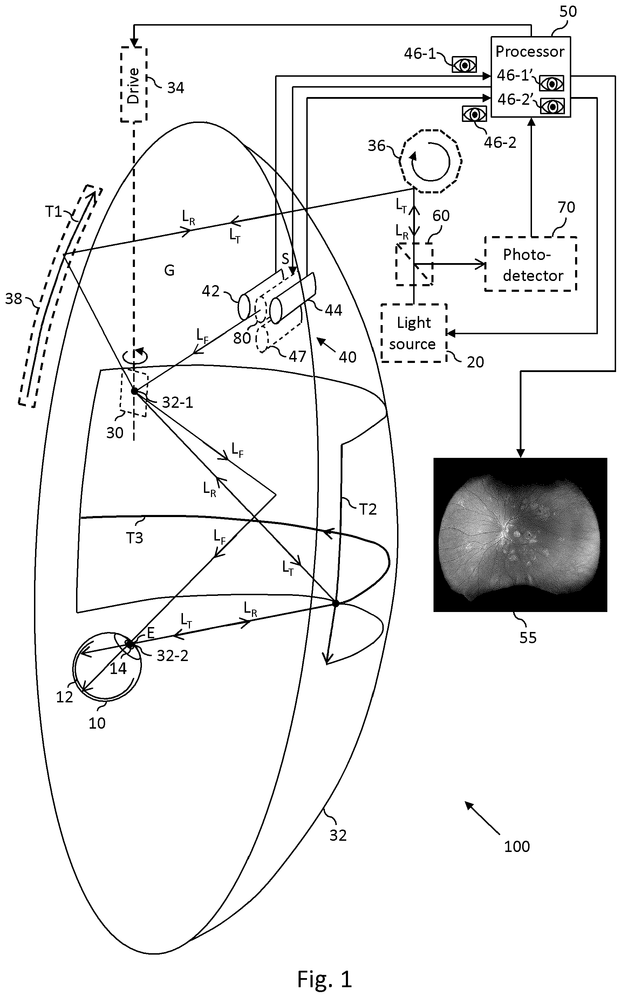

Figure 1 is a schematic illustration of an ophthalmic imaging instrument 100 according to the first example embodiment, which is operable to image a portion of an eye 10 of a subject. The ophthalmic imaging instrument 100 may, as in the present example embodiment, be arranged to image the portion of the eye 10 by scanning a beam of light across the eye 10, although the techniques described herein as not limited to scanning ophthalmic imaging instruments. The portion imaged may, as in the present example embodiment, comprise a region of a fundus 12 of the eye 10. The ocular fundus 12 is the inner lining of the eye 10 opposite the lens, and comprises the retina, macula, optic disc, fovea, choroid, and blood vessels. The ophthalmic imaging instrument 100 may additionally or alternatively be operable to image another portion of the eye 10, such as at least a part of the anterior segment of the eye 10.

The ophthalmic imaging instrument 100 may be a widefield (WF) ophthalmic imaging instrument, which is operable to acquire a WF image of the fundus 12. A WF image of the fundus 12 is defined as a single-capture image, centred on the fovea of the eye 10, which captures retinal anatomic features in the mid-periphery that are posterior to the vortex vein ampulla, in all four quadrants (i.e. superior, inferior, nasal and temporal) of the eye 10. A WF ophthalmic imaging instrument can thus acquire a single-capture image which covers a region of the retina extending from the fovea up to the posterior edge of vortex vein ampulla, and is defined to have a field of view (FoV) between 60 and 100 degrees. Here, the FoV is expressed in terms of the eye-angle, which is the angle subtended by the imaged retinal region at the spherical centre of the eye 10, i.e. the point of intersection between the vertical diameter of the eyeball and the visual axis.

The ophthalmic imaging instrument 100 may, as in the present example embodiment, be provided in form of an ultra-widefield (UWF) ophthalmic imaging instrument, which has a larger FoV than a WF instrument and is operable to acquire an UWF image of the ocular fundus 12 covering a larger proportion of the ocular fundus 12. An UWF image of the ocular fundus 12 is defined as a single-capture image, centred on the fovea of the eye 10, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

It should be noted that the techniques described herein are not limited in their applicability to WF and UWF ophthalmic imaging instruments but are also applicable to ophthalmic imaging instruments having a smaller FoV, and may benefit any ophthalmic imaging instrument which illuminates a portion of the eye 10 via one or more curved (e.g. ellipsoidal) mirrors to image the portion. However, the inventors have found that it is particularly important to compensate for the warping of stereo images caused by the curved mirror(s) in WF and especially UWF ophthalmic imaging instruments, as the consequences of not doing so when trying to bring the pupil within a target range for fundus image acquisition are particularly pronounced in these types of ophthalmic imaging instrument. As noted above, precise pupil alignment is particularly important in WF and UWF ophthalmic imaging instruments, owing to the acquisition of the WF/UWF fundus image relying on light propagating to and from the fundus, through the pupil, at widely varying angles of incidence on the pupillary plane, which is prone to being clipped by the pupil unless the pupil alignment is satisfactory.

In the present example embodiment, the ophthalmic imaging instrument 100 comprises an UWF combined scanning laser ophthalmoscope (SLO) and optical coherence tomography (OCT) instrument, which is arranged to acquire OCT images of the ocular fundus 12 using well-known interferometric imaging techniques, as well as fundus images of one or more additional modalities. Examples of such additional imaging modalities include pseudo-colour imaging, fundus autofluorescence (FAF), fluorescein angiography (FA), and indocyanine green angiography (ICGA). By way of an example, the Optos Monaco^{™} is a combined SLO-OCT system capable of acquiring green or red (or combined green and red) laser views, and green laser autofluorescence views, as well as OCT views. However, the ophthalmic imaging instrument 100 need not be a combined SLO-OCT system, and may instead be an OCT imaging instrument operable to acquire OCT images only, or an UWF SLO operable in one or more of the aforementioned (or other) SLO imaging modalities but not in an OCT modality. The Optos Daytona^{™} is an example of such a multi-modal UWF SLO.

Where the ophthalmic imaging instrument 100 is an ophthalmic imaging scanner, as in the present example embodiment, it may comprise a light source 20 arranged to emit at least one beam (understood to be any directional projection) of light L_{T} for imaging the eye 10, and an optical system comprising a rotatable mirror 30, for scanning the beam(s). The ophthalmic imaging instrument 100 comprises an ellipsoidal mirror 32, via which the ophthalmic imaging instrument 100 is arranged to acquire the UWF image of the ocular fundus 12, and a stereo imaging apparatus 40 (described in more detail below) to assist in bringing the eye 10 into a position at which it can be imaged by the ophthalmic imaging instrument 100. The optical system is arranged to scan the at least one beam of light L_{T} across the eye 10, and collect and guide return light L_{R} from the region of the eye 10 illuminated by the beam of light L_{T}. The return light L_{R} may be a portion of the beam of light L_{T} which has been reflected or scattered from the eye 10, or it may be light whose emission is stimulated or otherwise caused by the beam of light L_{T}, as in the case of the FAF and ICGA imaging modalities, for example.

The ophthalmic imaging instrument 100 further comprises a processor (or controller) 50, and may, as in the present example embodiment, also include a beam splitter 60 and a photodetector 70. The beam splitter 60 is arranged to split off a portion of the return light L_{R} and direct the split-off portion of the return light L_{R} to the photodetector 70. An UWF image 55 of the eye 10 is acquired by the ophthalmic imaging instrument 100 (for example, by the processor 50) by processing the output of the photodetector 70 using well-known techniques.

The optical system may also include a drive mechanism 34 comprising a galvanometer, for example, which is controlled by the processor 50 to cause the rotatable mirror 30 to scan the beam of light L_{T} by undergoing a predetermined rotational motion. This may, as in the present example embodiment, comprise a predetermined oscillatory rotational motion, wherein the rotatable mirror 30 rotates alternatively clockwise and anticlockwise by a predetermined angle (or, in other words, wherein the rotatable mirror 30 repeatedly rotates across an angular range defined about the mirror's axis of rotation, reversing its direction of rotation at each end of the angular range).

The light source 20 is, in general, arranged to generate light in one or more ranges of wavelength that are suitable for imaging the ocular fundus 12 (or other part of the eye 10, as the case may be), for example in the visible spectrum (e.g. red and green light) and/or the near-infrared spectrum. The light source 20 may, for example, comprise one or more laser diodes or one or more super-luminescent diodes (or a combination of one or more laser diodes and one or more super-luminescent diodes), and may also have one or more optical components, such as collimators, apertures and lenses, which are arranged to generate one or more light beams. The optical system (discussed further below) may be arranged to illuminate a region of the ocular fundus 12 with a (flying) spot of light or a line of light (in case of the ophthalmic imaging instrument 100 being a line-field (i.e. line-scanning) system) generated using a cylindrical lens or other well-known components or optical assemblies for generating line-field illumination.

In example embodiments like the present, wherein the ophthalmic imaging instrument 100 is operable in an OCT imaging mode, a swept light source (in case of the ophthalmic imaging instrument 100 being a swept-source OCT (SS-OCT) system) or a broadband source (in case of the ophthalmic imaging instrument 100 being a spectral-domain OCT (SD-OCT) system) may be provided for OCT imaging.

The form of the photodetector 70 is not limited and the photodetector 70 may, for example, comprise a balanced photodetector arrangement comprising two reverse-biased photodiodes whose output photocurrents are subtracted from one another, the subtracted current signal being converted to a voltage detection signal by a transimpedance amplifier. In example embodiments where the ophthalmic imaging instrument 100 is provided in the form of a line-scan SLO, the photodetector 70 may comprise a one- or two-dimensional array of photo-sensing elements. In example embodiments like the present, where the ophthalmic imaging instrument 100 features an OCT imaging modality, a spectrometer (where a SD-OCT set-up is used) or a photodiode detector (where a SS-OCT set-up is used) may be provided to detect interference light from the sample arm and reference arm of the interferometer.

The optical system may, as in the present example embodiment, be arranged to perform a two-dimensional point scan of the light L_{T} from the light source 20 across the region of the ocular fundus 12 that is illuminated by the point-scan, and to collect light from the illuminated region during the point-scan. The optical system may, as in the present example embodiment, be arranged to perform the two-dimensional point scan in part by the rotatable mirror 30 scanning the beam of light L_{T} across the ocular fundus 12 via the ellipsoidal mirror 32. Furthermore, by way of an example, the optical system further comprises a scanner 36 and a curved mirror 38, which are arranged to scan the beam of light L_{T} in a first direction across the ellipsoidal mirror 32 via the rotatable mirror 30. The scanner 36 may, as in the present example embodiment, be provided in the form of a polygon scanner comprising a plurality (e.g. 16) facets arranged around the circumference of a wheel which is rotated at a high speed (typically over 30,000 revolutions per minute) to perform high-frequency repeat scans of the light beam L_{T}. However, the scanner 36 may be provided in other forms, such as a galvanometer scanner, a micro-electromechanical system (MEMS) scanning mirror or a resonant scanning mirror, for example, or may be replaced by a cylindrical lens or other means of generating a beam comprising a "fan" of light rays whose projection onto a flat surface forms a line of light in case the optical system is a line-field system. Where the ophthalmic imaging instrument 100 has an OCT imaging modality, as in the present example embodiment, a second galvanometer scanner (not shown in Fig. 1) may be provided to scan the OCT sample beam in the first direction across the ellipsoidal mirror 32, via the rotatable mirror 30.

The light beam L_{T} is reflected, in sequence, by the scanner 36, the curved mirror 38, the rotatable mirror 30 and the ellipsoidal mirror 32, before being incident on the fundus 12 via the pupil 14 of the eye 10. Light from the illuminated region of the fundus 12 follows the same optical path through the optical system as the light beam that had entered the optical system but does so in reverse order, and a part thereof is directed to the photodetector 70 by the beam splitter 60.

The two-dimensional point scan is performed by the scanner 36 scanning the light beam L_{T} in a first (e.g. vertical) direction across the region of the ocular fundus 12, and by the rotatable mirror 30 rotating about its rotational axis to scan the light beam L_{T} in a second (e.g. horizontal) direction across the region of the fundus 12 (which second direction may, as in the present example embodiment, be orthogonal to the first direction). The ellipsoidal mirror 32 has a first focal point 32-1 and a conjugate second focal point 32-2 and may, as in the present example embodiment, take the form of a spheroidal mirror having an axis of rotational symmetry, specifically rotational symmetry about its major axis which passes through the focal points. More generally, any form of curved mirror having a curvature that enables it to transfer light rays incident on a first focal point of the curved mirror to a conjugate second focal point of the curved mirror may be employed in example embodiments as a generalisation of the ellipsoidal mirror 32. The ellipsoidal mirror 32 may be substantially spheroidal in some example embodiments, with some deviation in curvature from the spheroidal form that nevertheless allows the ophthalmic imaging instrument 100 to acquire WF or UWF images of the fundus 12. The rotational axis of the rotatable mirror 30 may, as in the present example embodiment, be parallel to the axis of circular symmetry of the spheroidal mirror. The length of the optical path between the first focal point 32-1, and points along a path T3 on the ellipsoidal mirror 32 followed by the beam of light L_{T} incident thereon as the beam is scanned by rotation of the rotatable mirror 30, is therefore constant. The scanning performed by the scanner 36 and the rotatable mirror 30 may be coordinated by the processor 50 or a scanning system controller (not shown) such that the beam of light L_{T} is scanned across the ocular fundus 12 in accordance with a predefined scan pattern.

The curved mirror 38 (also referred to as a slit mirror) may be an ellipsoidal mirror, as in the present example embodiment. Each of the curved mirror 38 and the ellipsoidal mirror 32 thus has a respective first focal point and a respective conjugate second focal point. The scanner 36 is arranged to reflect the beam of light L_{T} at the first focal point of the curved mirror 38, such that a projection of the beam L_{T} on the curved mirror 38 follows the trajectory T1 shown in Figure 1. The curved mirror 38 reflects the beam of light L_{T} incident thereon to its second focal point, and the rotatable mirror 30 (where provided) is arranged to reflect the beam of light L_{T} at the second focal point of the curved mirror 38 onto the ellipsoidal mirror 32. The rotatable mirror 30 may, as in the present example embodiment, also be arranged to reflect the beam L_{T} at the first focal point 32-1 of the ellipsoidal mirror 32, and a pupil 14 of the eye 10 is disposed at the second focal point 32-2 of the ellipsoidal mirror 32 during imaging of the eye 10. As the beam of light L_{T} is scanned across the curved mirror 38, the beam L_{T} is reflected therefrom onto the ellipsoidal mirror 32 via the rotatable mirror 30, such that a projection of the beam L_{T} on the ellipsoidal mirror 32 follows the trajectory T2 shown in Figure 1 during performance of each so-called "vertical scan" along a so-called "vertical" (or "fast") scan direction across the ellipsoidal mirror 32. As the rotatable mirror 30 rotates, it causes the adjacent vertical scans to be displaced from one another along a so-called "horizontal" (or "slow") scan direction, such that points in the vertical scans having the same elevation are arranged along the path T3 illustrated in Figure 1.

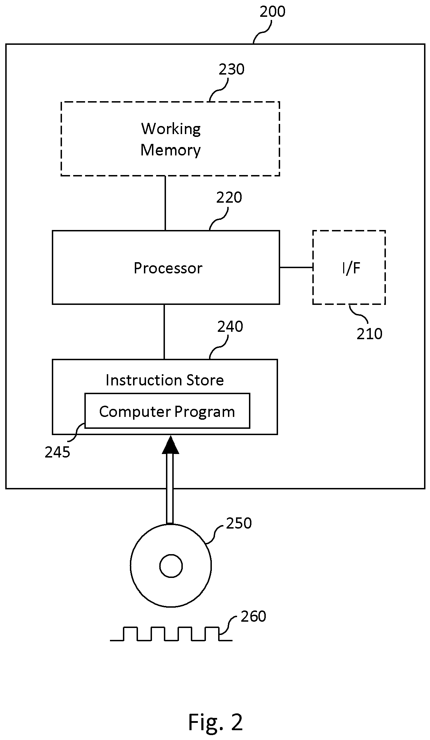

The processor 50 may be provided in any suitable form, for example as a processor 220 of a programmable signal processing hardware 200 of the kind illustrated schematically in Figure 2. The programmable signal processing hardware 200 comprises a communication interface (I/F) 210, for communicating control signals and/or data with the light source 20, the drive mechanism 34, the stereo imaging apparatus 40, the photodetector 70 and a fixation target light source, as described herein. The signal processing hardware 200 further comprises a processor 220 (e.g. a Central Processing Unit, CPU, and/or a Graphics Processing Unit, GPU), a working memory 230 (e.g. a random-access memory) and an instruction store 240 storing a computer program 245 comprising the computer-readable instructions which, when executed by the processor 220, cause the processor 220 to perform various functions of the processor 50 described herein.

The working memory 230 stores information used by the processor 220 during execution of the computer program 245. The instruction store 240 may comprise a ROM (e.g. in the form of an electrically erasable programmable read-only memory (EEPROM) or flash memory) which is pre-loaded with the computer-readable instructions. Alternatively, the instruction store 240 may comprise a RAM or similar type of memory, and the computer-readable instructions of the computer program 245 can be input thereto from a computer program product, such as a non-transitory, computer-readable storage medium 250 in the form of a CD-ROM, DVDROM, etc. or a computer-readable signal 260 carrying the computer-readable instructions. In any case, the computer program 245, when executed by the processor 220, causes the processor 220 to perform the functions of the processor 50 as described herein. More generally, the processor 50 of the present example embodiment may comprise one or more instances of the computer processor 220 and one or more instances of the memory 240 storing computer-readable instructions which, when executed by the computer processor(s) 220, cause the computer processor(s) 220 to perform the functions of the processor 50 as described herein. Where a plurality of processors 220 is provided, the processors 220 may communicate with each other via any kind of computer network.

It should be noted, however, that the processor 50 may alternatively be implemented in non-programmable hardware, such as an ASIC, an FPGA or other integrated circuit dedicated to performing the functions of the processor 50 described herein, or a combination of such non-programmable hardware and programmable hardware as described above with reference to Figure 2.

Referring again to Figure 1, the stereo imaging apparatus 40 may form part of a so-called pupil alignment module (PAM), and comprises a first camera 42 and a second camera 44 that are arranged to acquire respective stereo images 46-1 and 46-2 of the pupil 14 and a surrounding portion of the eye 10 (including at least some of the iris and typically also some of the sclera) via the ellipsoidal mirror 32 and optionally also via the rotatable mirror 30, as in the present example embodiment. It should be noted, however, that the stereo imaging apparatus 40 may acquire the stereo images 46-1 and 46-2 by detecting light from the eye 10 which has been reflected by the ellipsoidal mirror 32 directly onto the stereo imaging apparatus 40 via the first focal point 32-1, for example in a variant where the rotatable mirror 30 is moved away from the first focal point 32-1 in the patient alignment mode described below, and the stereo imaging apparatus 40 is positioned so that it can image the eye 10 directly via the ellipsoidal mirror 32 (i.e. without the rotatable mirror 30 reflecting any light that is used in the imaging). In such a variant, the fixation target light source 80 may be positioned to project fixation target light L_{F} into the fundus 12 directly via the ellipsoidal mirror 32 (i.e. without the rotatable mirror 30 reflecting the fixation target light L_{F}).

The stereo imaging apparatus 40 may, as in the present example embodiment, also have an illumination source 47 which illuminates the eye 10 via the ellipsoidal mirror 32 (and via the rotatable mirror 30, where provided) with light whose reflection from the eye 10 is detected by the first camera 42 and the second camera 44 to acquire the respective stereo images 46-1 and 46-2 of the pupil 14. The illumination is typically in the infra-red (IR) band for patient comfort and to avoid pupil constriction, and may be provided by an illumination source 47 in the form of an IR light-emitting diode (LED), for example. The stereo cameras 42 and 44 may employ complementary metal oxide semiconductor (CMOS) sensors, and fast lenses of fixed focal length to compensate for the sensors' relatively low sensitivity to the IR illumination that is typically provided by the illumination source. The ophthalmic imaging instrument 100 may, as in the present example embodiment, further comprise (e.g. as part of the PAM) a fixation target light source 80 comprising one or more light sources such as light-emitting diodes (LEDs), for example. The fixation target light source 80 is arranged to project fixation light L_{F} onto the fundus 12 via the rotatable mirror 30 and the ellipsoidal mirror 32. A projection of the fixation target light L_{F} on the fundus 12 has a predetermined shape chosen to assist the subject in maintaining a steady gaze direction, which may, for example, include one or more of a crosshair, a dot, a disc or a circle. The colour of the fixation light L_{F} may be variable by the processor 50, as described below.

The processor 50 may, as in the present example embodiment, be arranged to operate in a patient alignment mode to control the drive mechanism 34 to rotate the rotatable mirror 30 to a predetermined orientation (if the rotatable mirror 34 is not already in that orientation), and to hold the rotatable mirror 30 stationary in the predetermined orientation while controlling the stereo imaging apparatus 40 to acquire the stereo images 46-1 and 46-2 of the pupil 14 at the same time (or with a small delay between the acquisitions of the two images that does not significantly affect the stereoscopic distance measurement) via the rotatable mirror 30 and the ellipsoidal mirror 32. The processor 50 may more generally control the stereo imaging apparatus 40 to acquire the stereo image 46-1 and 46-2 via the first focal point 32-1 of the ellipsoidal mirror 32, in some cases without use of the rotatable mirror 30, for example as in the variant described above, where the rotatable mirror 30 is moved away from the first focal point 32-1 during operation of the processor 50 in the patient alignment mode, for example by a rotation mechanism arranged to rotate an assembly comprising the rotatable mirror 30, the drive 34 and a shaft connecting these components such that the rotatable mirror 30 moves away from the first focal point 32-1. The processor 50 may control the stereo imaging apparatus 40 to keep acquiring stereo images of the pupil 14 during operation in the patient alignment mode.

The processor 50 is further configured to process the acquired stereo images to generate a signal S for bringing the pupil 14 within a target range suitable for acquiring an UWF image 55 of the fundus 12. In more detail, the processor 50 is arranged to generate dewarped stereo images 46-1' and 46-2' of the pupil 14 by de-warping the stereo images 46-1 and 46-2, i.e. processing the stereo images 46-1 and 46-2 to compensate (i.e. at least partially correct for) for a warping in the stereo images 46-1 and 46-2 caused by a curvature of the ellipsoidal mirror 32, i.e. a geometric distortion of each stereo image caused by a departure from rectilinear projection that is provided by the ellipsoidal mirror 32, the departure resulting in straight lines on an imaging target, which is being imaged via the ellipsoidal mirror 32, being curved in the acquired image, for example. In the present example embodiment, the processor 50 is arranged to generate the dewarped stereo images 46-1' and 46-2' of the pupil 14 by using a pixel mapping, which is derived from an optical model of the ellipsoidal mirror 32, to transform the stereo images 46-1 and 46-2 to compensate for the warping of the stereo images 46-1 and 46-2 caused by the curvature of the ellipsoidal mirror 32. The optical model may be created in any suitable optical modelling software, such as Ansys Zemax OpticStudio^{™}, for example, using the known geometry of the ellipsoidal mirror 32. The optical modelling software may be used to calculate the distortion imparted on the stereo images 46-1 and 46-2, and the distortion may then be used to generate a mapping for de-warping the stereo images 46-1 and 46-2 to generate the de-warped stereo images 46-1' and 46-2'. The mapping may be used to re-map the stereo images 46-1 and 46-2, by translating each pixel in each of the stereo images 46-1 and 46-2 in an X- and/or a Y-direction of the image frame, in accordance with the mapping, to generate the respective de-warped image. However, the de-warping need not be performed in this way, and another approach may be taken instead.

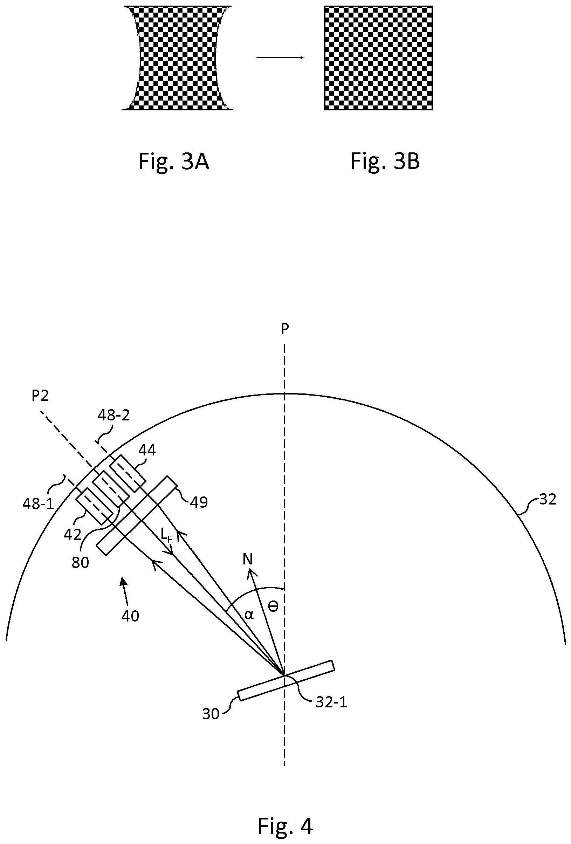

For example, an imaging target of a known shape may be used to measure the amount of distortion imparted on the stereo images 46-1 and 46-2 by the ellipsoidal mirror 32. The imaging target could, for example, be a chequerboard pattern of black and white squares, as illustrated in Figure 3B. A pixel mapping may then be generated and used to de-warp the distorted grid as acquired by the stereo imaging apparatus 40, which is illustrated schematically in Figure 3A, to generate a de-warped image showing the original chequerboard pattern shown in Figure 3B. This de-warping could also be performed by a supervised machine learning algorithm (e.g. a convolutional neural network), which has been trained using images of the kind shown in Figures 3A and 3B, for example, to learn the two-dimensional deformation field which can move pixels from the distorted input images to corresponding pixel locations in the undistorted images. A further alternative would be a hybrid of the above approaches, in which a model of the ellipsoidal mirror 32 is used to generate a generic mapping for an ideal geometry of the ellipsoidal mirror 32. An imaging target could then be used to perform calibration of the generic mapping for each copy of the UWF ophthalmic imaging instrument 100 during manufacture, to account for manufacturing tolerances in the ellipsoidal mirror 32. The de-warping may be performed in other ways, using approaches well-known in the field of digital image processing.

While the rotatable mirror 30 remains stationary in the predetermined orientation in the patient alignment mode (which may also be referred to herein as a fixation mode, or a patient alignment and fixation mode), the processor 50 is also arranged to control the fixation target light source 80 to project the fixation light L_{F} onto the rotatable mirror 30, specifically onto the first focal point 32-1 of the ellipsoidal mirror 32. The fixation target light source 80 is disposed in relation to the rotatable mirror 30 whilst in the predetermined orientation such that the fixation light L_{F} is projected onto the fundus 12 via the rotatable mirror 30 and the ellipsoidal mirror 32 to fix the gaze direction of the eye 10 and thus keep the eye 10 steady while the pupil 14 is brought within the target range suitable for acquiring an ultra-widefield image 55 of the fundus 12 on the basis of the signal S. The fixation target light source 80 may, as in the present example embodiment, be arranged to project the fixation light L_{F} onto the rotatable mirror 30 from a location such that, when the rotatable mirror 30 is in the predetermined orientation, the fixation light L_{F} is incident on the eye 10 in a direction for fixing the gaze direction of the eye 10 in a central gaze direction, which the subject adopts while looking straight ahead.

In the patient alignment mode, the processor 50 may, as in the present example embodiment, process the stereo images acquired by the stereo imaging apparatus 40 using any known stereoscopic ranging technique which uses the concept of parallax and triangulation to estimate distance, to determine an indication of a distance d between the pupil 14 of the eye 10 and a reference position of the ophthalmic imaging instrument 100, at which the pupil 14 is to be placed for acquiring the UWF image 55 of the ocular fundus 12 by the ophthalmic imaging instrument 100. The reference position may, as in the present example embodiment, be an exit pupil E of the ophthalmic imaging instrument 100. For example, the processor 50 may determine the indication of the distance d by processing each image of the acquired stereo images 46-1 and 46-2 to locate a respective pupil centre of the pupil in the image (e.g. using edge detection to determine an outline of the pupil, and fitting a circle to the outline to find the circle centre), determining a separation between the pupil centres in a common image frame (i.e. a single image frame comprising both of the pupil centres), and determining the indication of the distance d using the determined separation, which may be inversely related to the distance d. The processor 50 may then compare the determined indication of the distance d with pre-determined threshold(s) to determine whether the pupil 14 is within a predetermined range of distances from the exit pupil of the ophthalmic imaging instrument 100 that is acceptable for acquiring the UWF image 55, too close to the exit pupil position (i.e. on one side of the rang of distances) or too far from exit pupil position (i.e. on the other side of the range of distances).

The processor 50 may, as in the present example embodiment, then generate the signal S to control the fixation target light source 80 to output fixation light of a colour that is indicative of the comparison result. For example, the fixation target light source 80 may be controlled by the processor 50 to emit first fixation light (or first background light to the fixation target light, which is projected together with the fixation target light) of a first colour (e.g. blue) in case the distance between the exit pupil E of the ophthalmic imaging instrument 100 and the pupil 14 is greater than a threshold value demarcating the end of the range R and the pupil 14 is on a side of the range R further from the ophthalmic imaging instrument 100, to emit second fixation light (or second background light to the fixation target light, which is projected together with the fixation target light) of a second, different colour (e.g. red) in case the distance between the exit pupil E and the pupil 14 is greater than the threshold value and the pupil 14 is on the other side of the range R which is nearer to the ophthalmic imaging instrument 100, and to emit third fixation light (or third background light to the fixation target light, which is projected together with the fixation target light) of a yet further colour (e.g. green) in case the distance between the exit pupil E and the pupil 14 is smaller than the threshold value. Although references are made herein to the exit pupil position, any other reference point of the image acquisition apparatus 2, in the vicinity of which the pupil 14 needs to be positioned for the acquisition of the UWF image 55 of the fundus 12, may be used instead.

Additionally or alternatively, the processor 50 may generate the control signal S to control a speaker (not shown in Figure 1) to generate a sound (e.g. of a varying tone or spoken word(s)) which is indicative of the comparison result. For example, the speaker may be controlled by the processor 50 to generate a first (e.g. low-pitched) tone or a spoken indication, for example, in case the distance between the exit pupil E of the ophthalmic imaging instrument 100 and the pupil 14 is greater than the threshold value and the pupil 14 is on the side of the range R further from the ophthalmic imaging instrument 100, a different, second (e.g. high-pitched) tone or spoken indication in case the distance between the exit pupil E and the pupil 14 is greater than the threshold value and the pupil 14 is on the other side of the range R which is nearer to the ophthalmic imaging instrument 100, and a yet further (e.g. medium-pitched) tone or spoken indication in case the distance between the exit pupil E and the pupil 14 is smaller than the threshold value.

By the processing of each acquired pair of stereo images as described above, the subject may be guided to move the eye 10 backwards (away from the ophthalmic imaging instrument 100) or forwards (towards the ophthalmic imaging instrument 100), as appropriate, until the subject observes the fixation light to be green and/or hears the medium-pitched tone or spoken indication (as the case may be), informing the subject that their pupil 14 is sufficiently close to the exit pupil (or other reference point) of the ophthalmic imaging instrument 100 for the UWF imaging of the fundus 12 to begin.

Although the signal S is arranged to provide (e.g. visual and/or audio) feedback to the subject, to guide them to move their eye 10 until the pupil 14 is sufficiently close to the exit pupil (or other reference point) of the ophthalmic imaging instrument 100 for the imaging of the fundus 12 to begin, the signal S may instead be arranged to control the ophthalmic imaging instrument 100 to automatically move its reference point relative to the eye 10 until the reference point is sufficiently close to the pupil 14 for the imaging of the fundus 12 to begin. The signal S may, for example, be arranged to control a drive mechanism which is configured to move the ophthalmic imaging instrument 100, or a portion thereof comprising the curved mirror(s) that relay imaging light to and from the eye 10 via a focal point of the mirror(s), towards and away from the eye 10.

After the distance between the exit pupil position and the pupil 14 has been determined to be smaller than the threshold value, the processor 50 may, as in the present example embodiment, be arranged to start operating in a fundus imaging mode to control the ophthalmic imaging instrument 100 to acquire the UWF image 55 of the fundus 12. The processor 50 may do this by controlling the light source 20 to start emitting the beam of light L_{T}, and controlling the ophthalmic imaging instrument 100 to acquire the UWF image 55 of the fundus 12 by controlling the scanner 36 and the rotatable mirror 30 to scan the beam of light L_{T} across the fundus 12 via the ellipsoidal mirror 32. The processor 50 preferably controls the fixation target light source 80 to stop emitting the fixation light L_{F} (before or after the light source 20 starts emitting the beam of light L_{T}) to reduce artifacts and otherwise improve the quality of the UWF image 55. To reduce the risk of the eye 10 becoming misaligned with the ophthalmic imaging instrument 100, the processor 50 may, as in the present example embodiment, begin to operate in the fundus imaging mode automatically in response to determining that the distance d is smaller than the threshold value. However, in other example embodiments, this switch in the mode of operation may be instructed by an operator of the ophthalmic imaging instrument 100 in reaction to visual, audio and/or tactile feedback based on the signal S, for example by the operator clicking a mouse button or pressing a key on a computer keyboard communicatively coupled to the processor 50.

The stereo imaging apparatus 40 may, as in the present example embodiment, be located within a region G between the rotatable mirror 30 and the ellipsoidal mirror 32 which is not traversed by the beam of light L_{T} during acquisition of the UWF image 55 of the fundus 12. The fixation target light source 80 may, as in the present example embodiment, also be located within the region G. The stereo imaging apparatus 40 and fixation target light source 80 may thus be accommodated in a region within the bowl of the ellipsoidal mirror 32 which is not used for beam propagation. This arrangement may advantageously allow the ophthalmic imaging instrument 100 to be made more compact, by placing the stereo imaging apparatus 40 and the fixation target light source 80 in an otherwise unused region of the ophthalmic imaging instrument 100.

Furthermore, accommodating the stereo imaging apparatus 40 and the fixation target light source 80 in the region G allows for the option of orientating the rotatable mirror 30 in the patient alignment mode (i.e. choosing the predetermined orientation mentioned above) such that the rotatable mirror 30 starts rotating from the predetermined orientation as soon as the UWF ophthalmic imaging instrument 100 starts acquiring the UWF image 55 during operation of the processor 50 in the fundus imaging mode. A delay in the start of image acquisition, which would be caused by a rotation of the rotatable mirror 30 from the predetermined orientation used in the patient alignment mode to a (different) start orientation for image acquisition, may therefore be avoided, and the risk of the gaze direction of the eye 10 changing thus reduced. However, if this advantage is outweighed by the need to provide easy access to the stereo imaging apparatus 40 or the fixation target light source 80 for maintenance and/or adjustment, for example, the stereo imaging apparatus 40 and the fixation target light source 80 may instead be located outside the bowl of the ellipsoidal mirror 32 in some example embodiments, with the predetermined orientation of the rotatable mirror 30 used in the patient alignment mode being set to still allow the stereo imaging apparatus 40 to acquire the stereo images 46-1 and 46-2 of the pupil 14 via the rotatable mirror 30 and the ellipsoidal mirror 32 while the eye 10 remains fixated. For example, the stereo imaging apparatus 40 and the fixation target light source 80 may be located above the rim of the ellipsoidal mirror (similarly to the curved mirror 38 in Figure 1, for example) so as to each have a line of sight to the rotatable mirror 30. Alternatively, the stereo imaging apparatus 40 and the fixation target light source 80 may be located on the non-reflecting side of the ellipsoidal mirror 32, with one of more holes being provided in the ellipsoidal mirror 32 to allow light to pass through the ellipsoidal mirror 32 to the stereo imaging apparatus 40 and from the fixation target light source 80.

Regardless of whether the rotatable mirror 30 is orientated in the patient alignment mode to allow the rotatable mirror 30 to start rotating from the predetermined orientation when the UWF ophthalmic imaging instrument 100 starts acquiring the UWF image 55, it may be advantageous to locate the stereo imaging apparatus 40 between the rotatable mirror 30 and the ellipsoidal mirror 32 at a location which minimises a deviation from circularity of respective representations of the pupil 14 in the stereo images 46-1 and 46-2 of the pupil 14 acquired by the stereo imaging apparatus 40 when the rotatable mirror 30 is in the predetermined orientation. Such a location of the stereo imaging apparatus 40 to bring its viewpoint to be as close as possible to a central gaze direction of the eye 10, and thus allow the pupil 14 in the images 46-1 and 46-2 to be as circular as possible, may facilitate the process of locating the pupil centre and therefore an accurate calculation of the distance between the pupil 14 and the exit pupil of the ophthalmic imaging instrument 100.

It should be noted, however, that even where the stereo imaging apparatus 40 is located between the rotatable mirror 30 and the ellipsoidal mirror 32 at a location which minimises the deviation from circularity of respective representations of the pupil 14 in the stereo images 46-1 and 46-2, each of the stereo images 46-1 and 46-2 is still dewarped to correct for image distortions introduced by the ellipsoidal mirror 32 (alone or in combination with one or more other optical elements of the ophthalmic imaging instrument 100) so that the pupil in the stereo images 46-1 and 46-2 appears more circular. This may be particularly desirable where the processor 50 is configured to display these images on a display to the operator of the ophthalmic imaging instrument 100, for example as an aid for aligning the ophthalmic imaging instrument 100 with the pupil 14, as in the scheme disclosed in US 9,173,561 B2, the contents of which are incorporated herein in their entirety by cross-reference.

By way of an example, the ellipsoidal mirror 32 of the present example embodiment has a plane of symmetry P comprising the first focal point 32-1 and the second focal point 32-2, and a normal direction N of the rotatable mirror 30 at the first focal point 32-1 subtends a predetermined angle θ relative to the plane of symmetry P when the rotatable mirror 30 is in the predetermined orientation, as illustrated in Figure 4. For clarity, the sizes of angle θ and angle α discussed below have been exaggerated in Figure 4. The first camera 42 and the second camera 44 are arranged symmetrically about a second plane P2, which passes through the first focal point 32-1 and the second focal point 32-2. The first camera 42 and the second camera 44 may, as in the present example embodiment, comprise respective camera lenses having respective optical axes 48-1 and 48-2 that are parallel to each other and arranged equidistantly from, and on opposite sides of, the second plane P2. However, in other example embodiments, the optical axes 48-1 and 48-2 may both be within the second plane P2. Where the normal direction N of the rotatable mirror 30 and the second plane P2 subtend an angle α at the first focal point 32-1 which is less than two times as large as the predetermined angle θ, the deviation from circularity of respective representations of the pupil 14 in the stereo images 46-1 and 46-2 of the pupil 14 acquired using this arrangement was found to be small enough to allow satisfactory pupil alignment to be achieved.

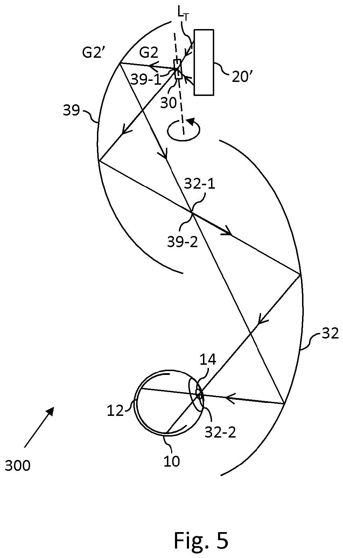

It should be noted that, although the rotatable mirror 30 is provided to reflect the beam of light L_{T} across the ellipsoidal mirror 32 at the first focal point 32-1 of the ellipsoidal mirror 32, the optical system of the ophthalmic imaging instrument 100 may be arranged such that the rotatable mirror 30 scans the beam L_{T} via the first focal point 32-1 in another way. Figure 5 is a schematic illustration of a line-scan UWF ophthalmic imaging instrument 300 according to a variant of the example embodiment which comprises, in addition to the ellipsoidal mirror 32, a second ellipsoidal mirror 39, via which the rotatable mirror 30 is arranged to scan a beam of light L_{T}' in the form of a line of light across the fundus 12. In this case, the beam of light L_{T}' has a projection onto a plane normal to its direction of propagation (i.e. a cross-section) which is a line, and may be generated from a beam of light having a cross-section of a spot (as generated by the light source 20) using a cylindrical lens, or may be generated using another optical arrangement for generating line-field illumination known to those versed in the art (e.g. a back-lit slit aperture). As illustrated in Figure 5, the second ellipsoidal mirror 39 has a first focal point 39-1 and a second focal point 39-2, with the rotatable mirror 30 being arranged to reflect the beam of light L_{T}' at the first focal point 39-1 of the second ellipsoidal mirror 39. The second focal point 39-2 of the second ellipsoidal mirror 39 coincides with the first focal point 32-1 of the ellipsoidal mirror 32.

The light source 20', which generates the line-field illumination L_{T}', is shown in Figure 5, and this is provided instead of the light source 20, the scanner 36 and the curved mirror 38 of the example embodiment of Figure 1. The line-scan UWF ophthalmic imaging instrument 300 includes the remaining components of the UWF ophthalmic imaging instrument 100, although these are not shown in Figure 5 to more clearly illustrate the different scan transfer arrangement comprising the two ellipsoidal mirrors 32 and 39. The line-scan UWF ophthalmic imaging instrument 300 also has a beam splitter to direct the return light from the fundus 12 to a photodetector for detecting the return line-field illumination, although this has similarly been omitted from Figure 5 for clarity.

In the line-scan UWF ophthalmic imaging instrument 300, the stereo imaging apparatus 40 and the fixation target light source 80 (where provided) may be accommodated in a region G2 (between the second ellipsoidal mirror 39 and the rotatable mirror 30) not traversed by the beam of light L_{T}'. The stereo imaging apparatus 40 and the fixation target light source 80 (where provided) may be arranged in relation to the rotatable mirror 30 and configured to operate as described above, although with the light propagating to the stereo imaging apparatus 40 from the eye 10, and the fixation light L_{F} propagating from the fixation target light source 80 to the eye 10, doing so via the second ellipsoidal mirror 39 as well as the ellipsoidal mirror 32.

Alternatively, if there is insufficient space within the bowl of the second ellipsoidal mirror 39, one or both of the stereo imaging apparatus 40 and the fixation target light source 80 may be located in a region G2' on the non-reflecting side of the second ellipsoidal mirror 39, with one or more holes being provided in the second ellipsoidal mirror 39 to allow light to pass through the second ellipsoidal mirror 29 to the stereo imaging apparatus 40 and from the fixation target light source 80.

Regardless of whether the stereo imaging apparatus 40 is accommodated within the region G (or region G2 in the variant of Figure 5), the stereo imaging apparatus 40 may, as in the present example embodiment, further comprise a Fresnel lens 49 disposed between the rotatable mirror 30 and both the first camera 42 and the second camera 44. In the example embodiment of Figure 1, the Fresnel lens 49 is arranged to refract light from the eye 10, which has been reflected by the rotatable mirror 30 at the first focal point 32-1 of the ellipsoidal mirror 32, to propagate along a first optical axis 48-1 of the first camera 42, and along a second optical axis 48-2 of the second camera 44. The Fresnel lens 49 allows the first camera 42 and the second camera 44 to be easily mounted on a supporting circuit board or other flat substrate, with their respective optical axes being normal to the surface of the substrate. The time-consuming process of carefully aligning the optical axes of the first camera 42 and the second camera 44 to pass through the first focal point 32-1 of the ellipsoidal mirror 32, which may otherwise need to be performed to improve the stereoscopic ranging, may be avoided, and the manufacture of the stereo imaging apparatus 40 consequently made simpler and faster. Similarly, in the variant described above with reference to Figure 5, the Fresnel lens 49 may be disposed between the rotatable mirror 30 and both the first camera 42 and the second camera 44 that are located in the region G2, and arranged to refract light from the eye 10, which has been reflected by the rotatable mirror 30 at the first focal point 39-1 of the second ellipsoidal mirror 39, to propagate along a first optical axis 48-1 of the first camera 42, and along a second optical axis 48-2 of the second camera 44.

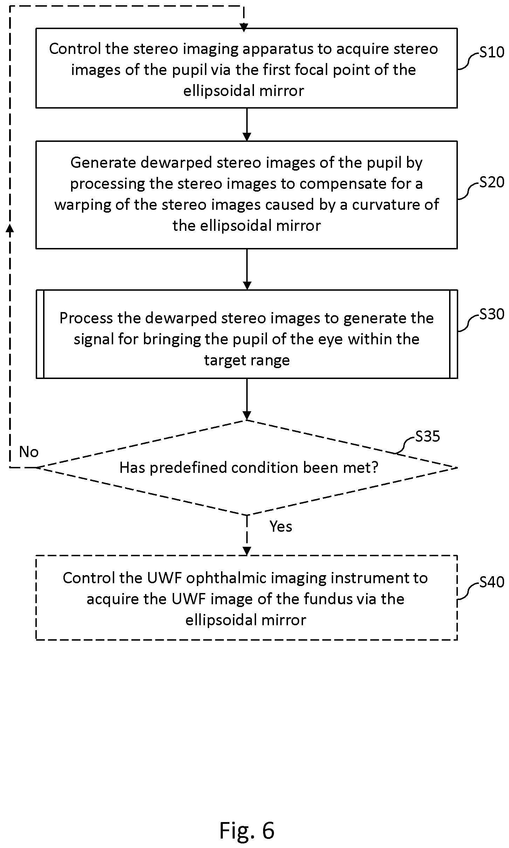

Figure 6 is a flow diagram summarising the operations performed by the processor 50 as described above to generate the signal S for bringing the pupil 14 within the target range for acquiring the UWF image 55 of the fundus 12 by the UWF ophthalmic imaging instrument 100 of the example embodiment. For clarity, some actions and processes that may be taken/performed in the context of these operations as described above will not be described again here but are understood to form optional features of the operations described in the following with reference to Figure 6. The processor 50 may similarly control the ophthalmic imaging instrument 300 of Figure 5 to bring the pupil 14 within the target range for acquiring an UWF image of the fundus 12.

In example embodiments like the present, where a rotatable mirror 30 is provided to scan the beam of light LT across the fundus 12 via the first focal point 32-1 of the ellipsoidal mirror 32, the processor 50 may begin operating in the patient alignment mode to generate the signal S to bring the pupil 14 of the eye 10 within a target range for acquiring the UWF image 55 of the fundus 12 by controlling the rotatable mirror 30 to be stationary in a predetermined orientation that allows the stereo imaging apparatus 40 to image the pupil 14 via the rotatable mirror 30 and the ellipsoidal mirror 32. The processor 50 may then control the illumination source of the stereo imaging apparatus 40 to turn on, thereby illuminating the eye 10 with IR light via the rotatable mirror 30 and the ellipsoidal mirror 32 (and via the second ellipsoidal mirror 39 in the variant of Figure 4).

In S10 of Figure 6, the processor 50 controls the stereo imaging apparatus 40 to acquire the stereo images 46-1 and 46-2 of the pupil 14 via the first focal point 32-1 of the ellipsoidal mirror 32. Where the rotatable mirror 30 is provided to scan the beam of light LT across the fundus 12 via the first focal point 32-1, as in the present example embodiment, the control of the stereo imaging apparatus 40 in S10 occurs while the rotatable mirror 30 is in the predetermined orientation. In the context of the variant of Figure 5, the processor 50 would control the stereo imaging apparatus 40 in S10 to acquire the stereo images 46-1 and 46-2 of the pupil 14 via the rotatable mirror 30, the second ellipsoidal mirror 39 and the ellipsoidal mirror 32, while the rotatable mirror 30 is in the predetermined orientation. Whilst the rotatable mirror 30 is in the predetermined orientation and the stereo imaging apparatus 40 is being controlled to acquire the stereo images 46-1 and 46-2 of the pupil 14, the processor 50 may (as an optional part of process S10 of Figure 6) control the fixation target light source 80 to project the fixation light L_{F} onto the rotatable mirror 30, the fixation target light source 80 being disposed in relation to the rotatable mirror 30 such that the fixation light L_{F} is projected onto the fundus 12 via the ellipsoidal mirror 32 to fix a gaze direction of the eye 10.

In S20 of Figure 6, the processor 50 generates dewarped stereo images 46-1' and 46-2' of the pupil 14 by processing the stereo images 46-1 and 46-2 to compensate for a warping in the stereo images 46-1 and 46-2 caused by a curvature of the ellipsoidal mirror 32. The processor 50 may generate the dewarped stereo images 46-1' and 46-2' of the pupil 14 by using a mapping, which is based on an optical model of the ellipsoidal mirror 32, to transform the stereo images 46-1 and 46-2 to compensate for the warping of the stereo images caused by the curvature of the ellipsoidal mirror 32, as described above.

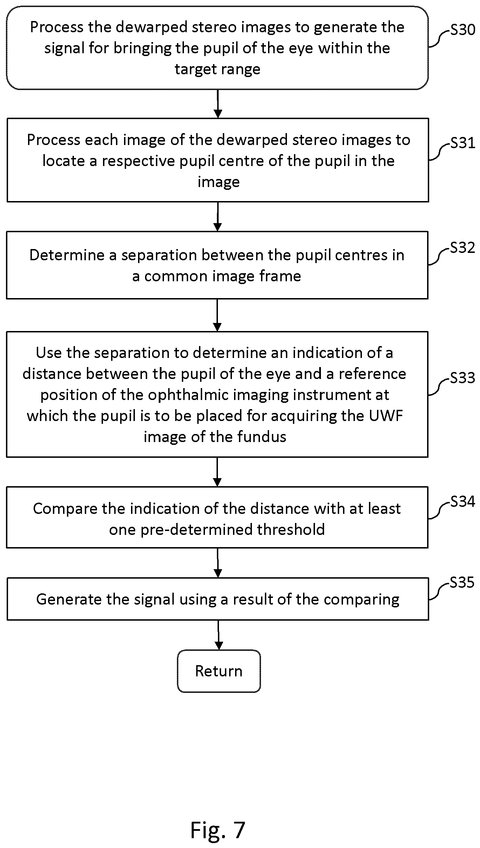

In S30 of Figure 6, the processor 50 processes the de-warped stereo images 46-1' and 46-2' of the pupil 14 to generate the signal S. The processor 50 may process the de-warped stereo images 46-1' and 46-2' to generate the signal S by the process described above, which is summarised in Figure 7.

In process S31 of Figure 7, the processor 50 processes each image of the dewarped stereo images 46-1' and 46-2' to locate a respective pupil centre of the pupil in the image. In S31, the processor 50 may process each image of the dewarped stereo images 46-1' and 46-2' to locate the respective pupil centre of the pupil in the image by using edge detection to determine an outline of the pupil in the image, and fitting a circle to the outline to determine, as the pupil centre in the image, a centre of the circle. Then, in process S32 of Figure 7, the processor 50 determines a separation between the pupil centres in a common image frame. In process S33 of Figure 7, the processor 50 uses the separation to determine an indication of a distance d between the pupil 14 of the eye 10 and a reference position of the ophthalmic imaging instrument 100 at which the pupil 14 is to be placed for acquiring the UWF image 55 of the fundus 12. In process S34 of Figure 7, the processor 50 compares the indication of the distance d with at least one pre-determined threshold. Then, in process S35 of Figure 7, the processor 50 generates the signal S using a result of the comparing in S34.

Referring again to Figure 6, in an optional process S35, the processor 50 may determine whether a predefined condition has been met. The predefined condition may, for example, be that the processor 50 has received an instruction provided by the operator of the ophthalmic imaging instrument 100, who is being provided with visual and/or audio feedback based on the signal S, to start imaging the fundus 12. As another example, the predefined condition may be that the distance between the reference point (e.g. exit pupil) of the ophthalmic imaging instrument 100 and the pupil 14 (as determined by the processor 50 based on the stereo images 46-1 and 46-2) is within the target range suitable for acquiring an UWF image 55 of the fundus 12. As a further alternative, the predefined condition may be that a predetermined time has elapsed from the start of operation of the processor 50 in the patient alignment mode after which the distance between the reference point (e.g. exit pupil) of the ophthalmic imaging instrument 100 and the pupil 14 is likely to be within the target range. If the processor 50 determines that the predefined condition has not been met, the process loops back to S10, otherwise it may proceed to S40 in Figure 6.

In S40 of Figure 6, the processor 50 controls the ophthalmic imaging instrument 100 to acquire the UWF image 55 of the fundus 12 via the ellipsoidal mirror 32. The processor 50 may, as in the present example embodiment, control the light source 20 to emit the beam of light L_{T}, and control the ophthalmic imaging instrument 100 to acquire the UWF image 55 of the fundus 12 by rotating the rotatable mirror 30 to scan the beam of light L_{T} across the fundus 12 via the ellipsoidal mirror 32 (and via the second ellipsoidal mirror 39 in the variant of Figure 4). Prior to the acquisition of the UWF image 55, the processor 50 preferably turns off the fixation target light source 80 and the IR illumination source 47.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the processor 50, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

## Claims

1. An ophthalmic imaging instrument (100; 300) arranged to acquire an image (55) of a fundus (12) of an eye (10), comprising:
an ellipsoidal mirror (32), via which the ophthalmic imaging instrument (100; 300) is arranged to acquire the image (55) of the fundus (12), the ellipsoidal mirror (32) having a first focal point (32-1) and a second focal point (32-2), wherein a pupil (14) of the eye (10) is positioned at the second focal point (32-2) during use of the ophthalmic imaging instrument (100);
a stereo imaging apparatus (40) arranged to acquire stereo images (46-1, 46-2) of the pupil (14) by detecting light from the eye (10) which has been reflected by the ellipsoidal mirror (32) via the first focal point (32-1) of the ellipsoidal mirror (32); and
a processor (50) arranged to generate, based on the stereo images (46-1, 46-2) of the pupil (14), a signal (S) for bringing the pupil (14) of the eye (10) within a target range for acquiring an image (55) of the fundus (12) by:
generating dewarped stereo images (46-1', 46-2') of the pupil (14) by processing the stereo images (46-1, 46-2) to compensate for a warping in the stereo images caused by a curvature of the ellipsoidal mirror (32); and
processing the dewarped stereo images (46-1', 46-2') to generate the signal (S) for bringing the pupil (14) of the eye (10) within the target range.

2. The ophthalmic imaging instrument (100) according to Claim 1, wherein the processor (50) is arranged to process the dewarped stereo images (46-1', 46-2') to generate the signal (S) by:
processing each image of the dewarped stereo images (46-1', 46-2') to locate a respective pupil centre of the pupil in the image;
determining a separation between the pupil centres in a common image frame;
using the separation to determine an indication of a distance (d) between the pupil (14) of the eye (10) and a reference position of the ophthalmic imaging instrument (100) at which the pupil (14) is to be placed for acquiring the image (55) of the fundus (12);
comparing the indication of the distance (d) with at least one pre-determined threshold; and
generating the signal (S) using a result of the comparing.

3. The ophthalmic imaging instrument (100) according to Claim 2, wherein the processor (50) is arranged to process each image of the dewarped stereo images (46-1', 46-2') to locate the respective pupil centre of the pupil in the image by using edge detection to determine an outline of the pupil in the image, and fitting a circle to the outline to determine, as the pupil centre of the pupil in the image, a centre of the circle.

4. The ophthalmic imaging instrument (100) according to any preceding claim, wherein the processor (50) is arranged to generate the dewarped stereo images (46-1', 46-2') of the pupil (14) by using a mapping, which is based on an optical model of the ellipsoidal mirror (32), to transform the stereo images (46-1, 46-2) to compensate for the warping in the stereo images caused by the curvature of the ellipsoidal mirror (32).

5. The ophthalmic imaging instrument (100) according to any preceding claim, wherein the ophthalmic imaging instrument (100; 300) is arranged to acquire the image (55) of the fundus (12) by scanning a beam of light (L_{T}) across the fundus (12), and further comprises:
a light source (20; 20') arranged to emit the beam of light (L_{T}; L_{T}'); and
a rotatable mirror (30) arranged to scan the beam of light (L_{T}; L_{T}') across the fundus (12) via the first focal point (32-1) of the ellipsoidal mirror (32) and via the ellipsoidal mirror (32), wherein
the stereo imaging apparatus (40) is arranged to acquire the stereo images (46-1, 46-2) of the pupil (14) via the rotatable mirror (30) and the ellipsoidal mirror (32),
the processor (50) is arranged to operate in a patient alignment mode to generate the signal (S), and to subsequently operate in a fundus imaging mode to control the ophthalmic imaging instrument (100) to acquire the image (55) of the fundus (12),
in the patient alignment mode, the processor (50) is arranged to:
control the rotatable mirror (30) to be stationary in a predetermined orientation; and
control the stereo imaging apparatus (40) to acquire the stereo images (46-1, 46-2) of the pupil (14) while the rotatable mirror (30) is stationary in the predetermined orientation, and
in the fundus imaging mode, the processor (50) is arranged to:
control the light source (20; 20') to emit the beam of light (L_{T}; L_{T}'); and
control the ophthalmic imaging instrument (100; 300) to acquire the image (55) of the fundus (12) by controlling the rotatable mirror (30) to scan the beam of light (L_{T}; L_{T}') across the fundus (12) via the ellipsoidal mirror (32).

6. The ophthalmic imaging instrument (100) according to Claim 5, wherein the rotatable mirror (30) is arranged to reflect the beam of light (L_{T}) at the first focal point (32-1), and the stereo imaging apparatus (40) is located within a region (G) between the rotatable mirror (30) and the ellipsoidal mirror (32) not traversed by the beam of light (L_{T}) during acquisition of the image (55) of the fundus (12).

7. The ophthalmic imaging instrument (100) according to Claim 5 or Claim 6, wherein the stereo imaging apparatus (40) comprises:
a first camera (42) having a first optical axis (48-1), and a second camera (44) having a second optical axis (48-2) which is parallel to the first optical axis (48-1); and
a Fresnel lens (49) disposed between the rotatable mirror (30) and both the first camera (42) and the second camera (44), the Fresnel lens (49) being arranged to refract light from the eye (10), which has been reflected by the rotatable mirror (30), to propagate along the first optical axis (48-1) and the second optical axis (48-2).

8. The ophthalmic imaging instrument (100) according to any of Claims 5 to 7, further comprising:
a fixation target light source (80) arranged to project fixation light (L_{F}) onto the fundus (20) via the rotatable mirror (30) and the ellipsoidal mirror (32),
wherein, in the patient alignment mode, the processor (50) is arranged to control the fixation target light source (80) to project the fixation light (L_{F}) onto the rotatable mirror (30) in the predetermined orientation, the fixation target light source (80) being disposed in relation to the rotatable mirror (30) such that the fixation light (L_{F}) is projected onto the fundus (12) via the ellipsoidal mirror (32) to fix a gaze direction of the eye (10).

9. The ophthalmic imaging instrument (100) according to Claim 8 when dependent on Claim 6, wherein the fixation target light source (80) is located within the region (G) between the rotatable mirror (30) and the ellipsoidal mirror (32) not traversed by the beam of light (L_{T}) during acquisition of the image (55) of the fundus (12).

10. The ophthalmic imaging instrument (100) according to Claim 9, wherein the predetermined orientation is such that the rotatable mirror (30) starts rotating from the predetermined orientation when the ophthalmic imaging instrument (100) starts acquiring the image (55) of the fundus (12).

11. The ophthalmic imaging instrument (100) according to any preceding claim, wherein the ophthalmic imaging instrument (100) comprises at least one of an ultra-widefield, UWF, ophthalmic imaging instrument (100) arranged to acquire an UWF image (55) of the fundus (12), or a widefield, WF, ophthalmic imaging instrument arranged to acquire a WF Image of the fundus (12).

12. A method of generating a signal (S) for bringing a pupil (14) of an eye (10) within a target range for acquiring an image (55) of a fundus (12) of the eye (10) by an ophthalmic imaging instrument (100; 300), the ophthalmic imaging instrument (100; 300) comprising:
an ellipsoidal mirror (32), via which the ophthalmic imaging instrument (100; 300) is arranged to acquire the image (55) of the fundus (12), the ellipsoidal mirror (32) having a first focal point (32-1) and a second focal point (32-2), wherein the pupil (14) of the eye (10) is positioned at the second focal point (32-2) during use of the ophthalmic imaging instrument (100); and
a stereo imaging apparatus (40) arranged to acquire stereo images (46-1, 46-2) of the pupil (14) by detecting light from the eye (10) which has been reflected by the ellipsoidal mirror (32) via the first focal point (32-1) of the ellipsoidal mirror (32),
the method comprising:
controlling (S10) the stereo imaging apparatus (40) to acquire the stereo images (46-1, 46-2) of the pupil (14) via the first focal point (32-1) of the ellipsoidal mirror (32);
generating (S20) dewarped stereo images (46-1', 46-2') of the pupil (14) by processing the stereo images (46-1, 46-2) to compensate for a warping in the stereo images caused by a curvature of the ellipsoidal mirror (32); and
processing (S30) the dewarped stereo images (46-1', 46-2') to generate the signal (S) for bringing the pupil (14) of the eye (10) within the target range.

13. The method according to Claim 12, wherein the dewarped stereo images (46-1', 46-2') are processed (S30) to generate the signal (S) by:
processing (S31) each image of the dewarped stereo images (46-1', 46-2') to locate a respective pupil centre of the pupil in the image;
determining (S32) a separation between the pupil centres in a common image frame;
using (S33) the separation to determine an indication of a distance (d) between the pupil (14) of the eye (10) and a reference position of the ophthalmic imaging instrument (100) at which the pupil (14) is to be placed for acquiring the image (55) of the fundus (12);
comparing (S34) the indication of the distance (d) with at least one pre-determined threshold; and
generating (S35) the signal (S) using a result of the comparing.

14. The method according to Claim 13, wherein each image of the dewarped stereo images (46-1', 46-2') is processed (S31) to locate the respective pupil centre of the pupil in the image by using edge detection to determine an outline of the pupil in the image, and fitting a circle to the outline to determine, as the pupil centre in the image, a centre of the circle.

15. The method according to any of Claims 12 to 14, wherein the dewarped stereo images (46-1', 46-2') of the pupil (14) are generated (S20) by using a mapping, which is based on an optical model of the ellipsoidal mirror (32), to transform the stereo images (46-1, 46-2) to compensate for the warping in the stereo images caused by the curvature of the ellipsoidal mirror (32).
